# EUROPEAN PATENT APPLICATION

(11) **EP 3 878 374 A1**
(43) Date of publication of application: **15.09.2021**
(21) Application number: 19882103.5
(22) Date of filing: 31.10.2019
(51) Int. Cl.: A61B 17/00

(54) **OCCLUDER**

(30) Priority: 08.11.2018 CN 201811326853
(71) Applicant: Lifetech Scientific (Shenzhen) Co., Ltd, Shenzhen, Guangdong 518057 (CN)
(72) Inventor: CHEN, Xianmiao, Shenzhen, Guangdong 518057 (CN)
(74) Representative: J A Kemp LLP
(86) International application number: PCT/CN2019/114703
(87) International publication number: WO 2020/093925

(57) **Abstract**

An occluder, including an occlusion unit and an occlusion head connected to the occlusion unit. The occlusion unit has a central axis passing through a distal end and a proximal end of the occluder; the occluder further includes a marking structure, the marking structure includes a distal marker and at least two first markers, the distal marker is provided on the occlusion head, all the first markers located in the same placement plan are connected to the occlusion unit, the placement plane is perpendicular to the central axis, the distal marker is located outside the placement plane, projection points of all the first markers in a projection plane perpendicular to the placement plane is located on a straight line segment, and when the occluder is in a natural state, connection lines between the distal marker and two end points of the straight line segment form a triangle.

## Description

### Field

The embodiments relate to the field of medical devices, and more particularly relates to occluders.

### Background

In 1974, King and Mills completed transcatheter interventional therapy of secundum atrial septal defect (ASD) for the first time. Then, with the continuous improvement of occlusion devices, and particularly with the appearance of Amplatzer nitinol braided occluders since 1997, the nitinol braided occluders have been widely used in interventional treatment of congenital heart diseases such as atrial septal defect (ASD), ventricular septal defect (VSD), patent ductus arteriosus (PDA) and patent foramen ovale (PFO). At the present, transcatheter intervention of an occluder for minimally invasive treatment of ASD, VSD, PDA, PFO and other congenital heart diseases has become an important method. After a heart defect part is occluded, tissue surrounding the defect grows inward and completes the endothelialization of the occluder. The non-degradable occluder still exists in the defect part for a long time after the endothelialization, and easily causes long-term complications and adverse effects, including atrioventricular block, valve injury, residual shunt, heart abrasion, nickel allergy and the like. Therefore, a degradable occluder made of degradable materials is the latest development direction in the field of interventional occlusion. After the degradable occluder is implanted into a human body, an effect of occluding a defect can be achieved. After occlusion is completed, the materials forming the occluder are degraded in the body, and finally are metabolized into CO2 and water by the body and eliminated from the body, so that the human body is prevented from being affected by foreign matters for a long time.

Biodegradable materials used in the field of interventional therapy include, but are not limited to, polylactic acid (PLA), polyglycolic acid (PGA), polylactic-co-glycolic acid (PLGA), polyhydroxyalkanoate (PHA), polydioxanone (PDO), or polycaprolactone (PCL), and the like. The interventional occlusion is usually performed under the guidance of X-ray imaging equipment such as a Digital Subtraction Angiography (DSA), so the occluder is required to be radiopaque under X-ray, and thus be identifiable under the X-ray. The occluder made of the degradable material has poor developing performance under the X-ray, so it is difficult to determine its exact position and release state under the DSA, which leads to the decrease of success rate of implantation.

### Summary

Based on this, it is necessary to provide an occluder to solve the problem that the existing occluders are difficult to be determined an accurate position and a release state under X-ray.

An occluder includes an occlusion unit and an occlusion head connected to the occlusion unit. The occlusion unit has a central axis, and the central axis passes through a distal end and a proximal end of the occluder; the occluder further includes a marking structure; the marking structure includes a distal marker and at least two first markers; the distal marker is disposed on the occlusion head; all the first markers are connected to the occlusion unit; all the first markers are located in the same placement plane; the placement plane is perpendicular to the central axis; the distal marker is located outside the placement plane; projection points of all the first markers in a projection plane perpendicular to the placement plane are located on a straight line segment; and when the occluder is in a natural state, connection lines between the distal marker and two end points of the straight line segment form a triangle.

It can be understood that the occlusion unit includes a first sub-unit; the first sub-unit includes a first mesh disk and a first flow blocking film disposed in the first mesh disk; the central axis passes through a geometric center of the first mesh disk, and the central axis is perpendicular to a plane where the first mesh disk is located; the central axis passes through the geometric center of the first flow blocking film, and the central axis is perpendicular to a plane where the first flow blocking film is located; and all the first markers are connected to the first mesh disk, or all the first markers are connected to the first flow blocking film.

It can be understood that all the first markers are fixed on the first mesh disk; and when the occluder is in the natural state, the distance between each first marker and the central axis is equal to or less than the distance from an edge of the first mesh disk to the geometric center of the first mesh disk.

It can be understood that all the first markers are fixed on the first flow blocking film; and when the occluder is in the natural state, the distance between at least one of the first markers and the central axis is equal to the distance from an edge of the first flow blocking film to the geometric center of the first flow blocking film, or the distance between each first marker and the central axis is less than the distance from the edge of the first flow blocking film to the geometric center of the first flow blocking film.

It can be understood that when the occluder is in the natural state, the distance between each first marker and the central axis is less than the distance from the edge of the first flow blocking film to the geometric center of the first flow blocking film; all the first markers are distributed along a first circumference; and the first circumference and the first flow blocking film are concentrically disposed.

It can be understood that all the first markers are distributed equidistantly along the first circumference, and the number of the first markers ranges from 2 to 12.

It can be understood that the occlusion unit further includes a second sub-unit connected to the first sub-unit; the second sub-unit includes a second mesh disk and a second flow blocking film disposed in the second mesh disk; the central axis passes through a geometric center of the second mesh disk, and the central axis is perpendicular to a plane where the second mesh disk is located; the central axis passes through the geometric center of the second flow blocking film, and the central axis is perpendicular to a plane where the second flow blocking film is located; the marking structure further includes at least two second markers; and all the second markers are connected to the second mesh disk, or all the second markers are connected to the second flow blocking film.

It can be understood that all the second markers are fixed on the second mesh disk; and when the occluder is in the natural state, the distance between each second markers and the central axis is equal to or less than the distance from an edge of the second mesh disk to the geometric center of the second mesh disk.

It can be understood that all the second markers are fixed on the second flow blocking film; and when the occluder is in the natural state, the distance between at least one of the second markers and the central axis is equal to the distance from an edge of the second flow blocking film to the geometric center of the second flow blocking film, or the distance between each second marker and the central axis is less than the distance from the edge of the second flow blocking film to the geometric center of the second flow blocking film.

It can be understood that when the occluder is in the natural state, the distance between each second marker and the central axis is less than the distance from the edge of the second flow blocking film to the geometric center of the second flow blocking film; all the second markers are distributed along a second circumference; the second circumference and the second flow blocking film are concentrically disposed.

It can be understood that all the second markers are distributed equidistantly along the second circumference, and the number of the second markers ranges from 2 to 12.

It can be understood that the occlusion unit further includes a waist portion and a third flow blocking film; two ends of the waist portion are respectively connected to the first mesh disk and the second mesh disk; the third flow blocking film is disposed in the waist portion; the central axis passes through a geometric center of the third flow blocking film, and the central axis is perpendicular to a plane where the third flow blocking film is located; the marking structure further includes at least two third markers; and all the third markers are connected to the waist portion, or all the third markers are connected to the third flow blocking film.

It can be understood that all the third markers are connected to the third flow blocking film; and when the occluder is in the natural state, the distance between at least one of the third markers and the central axis is equal to a distance from an edge of the third flow blocking film to a geometric center of the third flow blocking film, or, the distance between each third marker and the central axis is less than the distance from the edge of the third flow blocking film to the geometric center of the third flow blocking film.

It can be understood that the number of the first markers, the number of the second markers and the number of the third markers are all equal to 2; and when the occluder is in the natural state, the two first markers are located at two ends of the same diameter of the first flow blocking film, the two second markers are located at two ends of the same diameter of the second flow blocking film, and the two third markers are located at two ends of the same diameter of the third flow blocking film; and the two first markers, the two second markers and the two third markers are coplanar.

The marking structure of the above occluder includes the distal marker and the at least two first markers, the distal marker is provided on the occlusion head, all the first markers are connected to the occlusion unit, all the first markers are located in the same placement plane, the placement plane is perpendicular to the central axis, the distal marker is disposed outside the placement plane, projection points of all the first markers in the projection plane perpendicular to the placement plane being located on a straight line segment; and when the occluder is in the natural state, connection lines between the distal marker and the two end points of the straight line segment form a triangle. In a release process of the occluder, the marking structure can be developed under the DSA to guide the release of the occluder. In a surgery, the exact position and the release state of the occlusion unit may be accurately determined with a morphologic change of the triangle formed by the connection lines between the distal marker and the two end points of the straight line segment, thereby increasing the success rate of the operation.

### Brief Description of the Drawings

Fig. 1 is a schematic structural diagram of an occluder in one embodiment.
Fig. 2 is a schematic structural diagram of cooperation between an occluder and an atrial septum in one embodiment.
Fig. 3 is a schematic structural diagram of cooperation between an occluder and a sheath in one embodiment.
Fig. 4a is a first state diagram of an occluder in one embodiment.
Fig. 4b is a second state diagram of an occluder in one embodiment.
Fig. 4c is a third state diagram of an occluder in one embodiment.
Fig. 4d is a fourth state diagram of an occluder in one embodiment.
Fig. 5 is a schematic structural diagram of a first flow blocking film and first markers in one embodiment.
Fig. 6 is a schematic structural diagram of an occlusion head, an occlusion unit, and a marking structure in one embodiment.
Fig. 7 is a top view of an occlusion unit in one embodiment.

### Detailed Description of the Embodiments

In order to make the above objectives, features and advantages of the embodiments more understandable, specific implementations of the embodiments are described in detail below in conjunction with the accompanying drawings. In the following description, numerous specific details are set forth in order to provide a thorough understanding of the embodiments. However, the embodiments can be embodied in many different forms from those herein set forth, and those of ordinary skill in the art can make similar improvements without departing from the spirit of the embodiments, so that the embodiments are not limited by the specific implementations below.

It should be noted that when one element is referred to as being "fixed" or "disposed" to another element, it can be directly on the other element or an intermediate element may also be present. When one element is referred to as being "connected" to another element, it can be directly connected to the other element or an intermediate element may also be present. The terms "perpendicular", "horizontal", "left", "right" and similar representations as used herein are for illustrative purposes only and are not meant to be the only implementations.

Unless otherwise defined, all terms used herein have the same meaning as common understandings of those of ordinary skill in the art to which the embodiments belong. The terms used in the description herein are for the purpose of describing the embodiments only and is not intended as limiting. As used herein, the term "and/or" includes any and all combinations of one or more of associated listed items.

In order to more clearly describe the structure of the embodiments, the terms "distal end" and "proximal end" are used as localizers which are conventional in the field of interventional medical devices. The "distal end" denotes an end away from an operator during an operation, and "proximal end" denotes an end close to an operator during an operation.

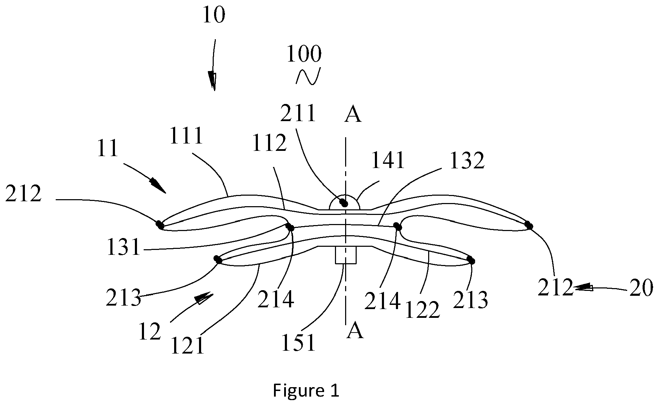

As shown in Fig. 1, the present embodiment provides an occluder 100, including an occlusion unit 10 and an occlusion head 141 connected to the occlusion unit 10. The occlusion unit 10 has a central axis A-A, the central axis A-A passes through a distal end and a proximal end of the occluder 100. The occluder 100 further includes a marking structure 20. The marking structure 20 includes a distal marker 211 and at least two first markers 212. The distal marker 211 is provided on the occlusion head 141. All the first markers 212 are connected to the occlusion unit 10. All the first markers 212 are located in the same placement plane, and the placement plane is perpendicular to the central axis A-A. The distal marker 211 is located outside the placement plane. Projection points of all the first markers 212 in a projection plane perpendicular to the placement plane are located on a straight line segment; and when the occluder 100 is in a natural state, connection lines between the distal marker 211 and two end points of the straight line segment form a triangle.

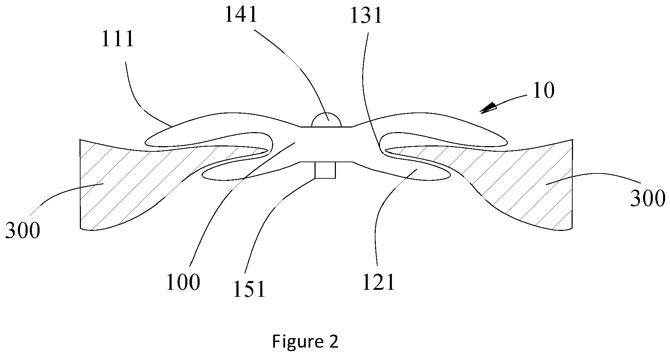

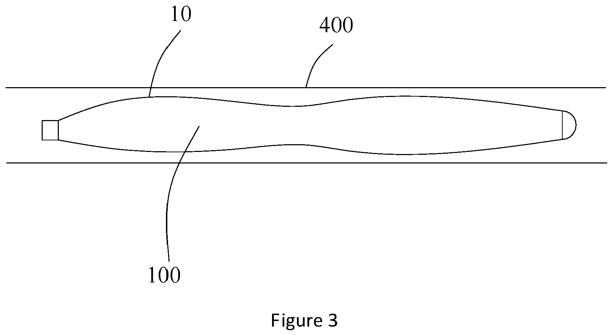

The occluder 100 of the present embodiment is used for occluding a defect of the atrial septum 300, as shown in Fig. 2. Referring to Fig. 3 together, the occluder 100 needs to be loaded into a conveyor before use. The conveyor includes a sheath 400 having an inner diameter of 2 to 5 mm. During implantation of the occluder 100, a surgical operation needs to be performed under the guidance of X-ray imaging equipment such as digital subtraction angiography (DSA), so that the occluder 100 can be conveyed to a distal end along the sheath 400, and after being conveyed to a related lesion part, the occluder 100 is applied with an acting force to release the occluder 100 from the sheath 400 to the atrial septum 300 to occlude a defect of the atrial septum 300. Specifically, after the sheath 400 loaded with the occluder 100 passes through the atrial septum 300, the acting force is applied to the occluder 100 to cause the occlusion unit 10 to move out of the sheath 400, and the occlusion unit 10 expands under the action of its own elastic force, thereby occluding the defect.

The marking structure 20 of the occluder 100 includes the distal marker 211 and the at least two first markers 212. The distal marker 211 is provided on the occlusion head 141. All the first markers 212 are connected to the occlusion unit 10. All the first markers 212 are located in the same placement plane, and the placement plane is perpendicular to the central axis A-A. The distal marker 211 is arranged outside the placement plane, and the projection points of all the first markers 212 in the projection plane perpendicular to the placement plane are located on a straight line segment. When the occluder 100 is in a natural state, the connection lines between the distal marker 211 and the two end points of the straight line segment form a triangle. When the occluder 100 is released, the marking structure 20 can be developed under the DSA to guide the release of the occluder 100.

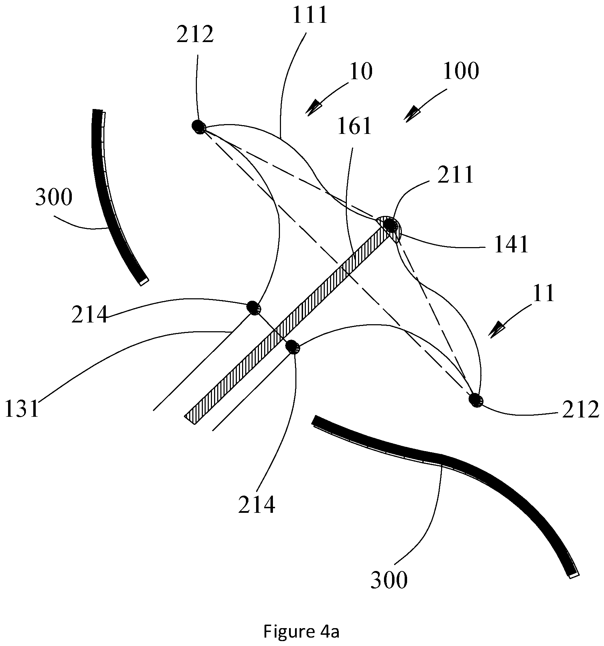

For example, as shown in Fig. 4a, an acting force is applied to the occluder 100 to cause the expansion of the occluder 100in the left atrium (the occluder 100 expanding in the left atrium is taken as an example here for description, but it is not limited to this). Parameters, such as a probe angle, of the DSA are adjusted to cause the projection points of all the first markers 212 in the projection plane perpendicular to the placement plane in a picture detected by the DSA to be located on one straight line segment and to cause the connection lines between the distal marker 211 and the two end points of the straight line segment to form a triangle. At this moment, a surgeon can apply an acting force to the occluder 100 to make it do reciprocating motion. It is observed whether the triangle deforms in the reciprocating motion process. It may be determined that the occlusion unit 10 is not adhered to the atrial septum 300 if the triangle does not deform in this process.

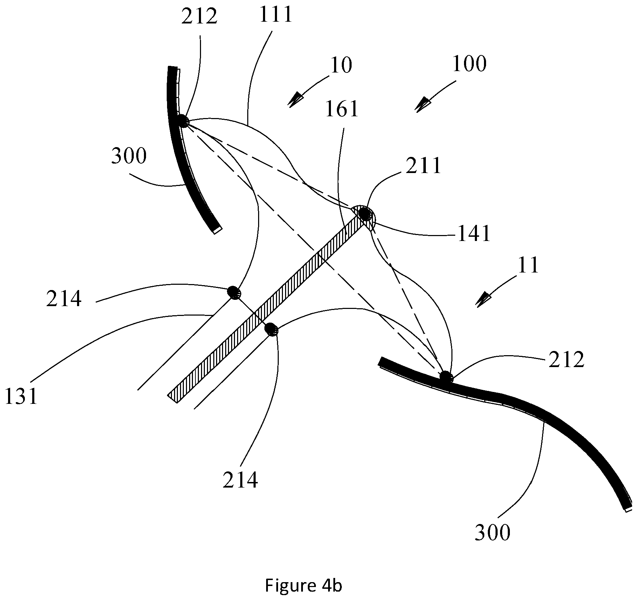

As shown in Fig. 4b, the occlusion unit 10 is continuously applied with an acting force, so that the occlusion unit 10 moves towards a proximal end till all the first markers 212 are adhered to the atrial septum 300. In this process, the surgeon is guided to do an operation by observing a geometric shape of the triangle and is assisted in learning a position relationship between the occlusion unit 10 and the atrial septum 300, so as to facilitate subsequent operations on the occluder 100. For example, after all the first markers 212 are adhered to the atrial septum 300, the motions of all the first markers 212 are limited, but the distal marker 211 can move freely. At this moment, the distal marker 211 continues to move closer to the atrial septum 300 and the first markers 212, and the triangle may deform. At this moment, it may be determined that the occlusion unit 10 has been adhered to the atrial septum 300.

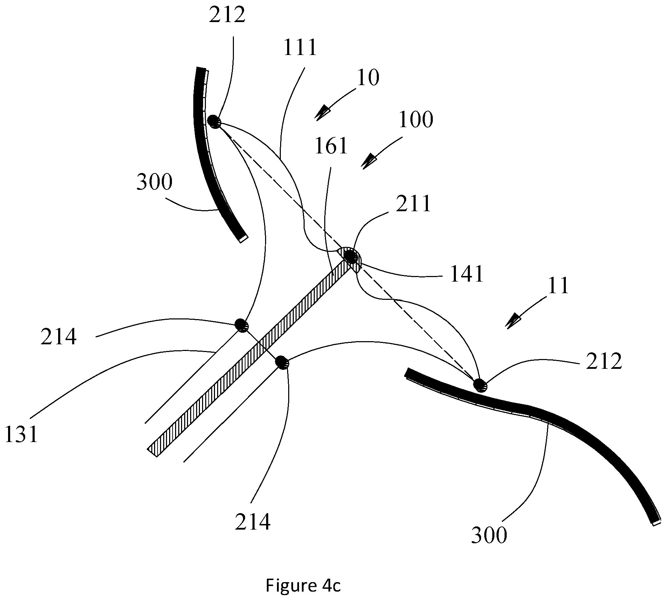

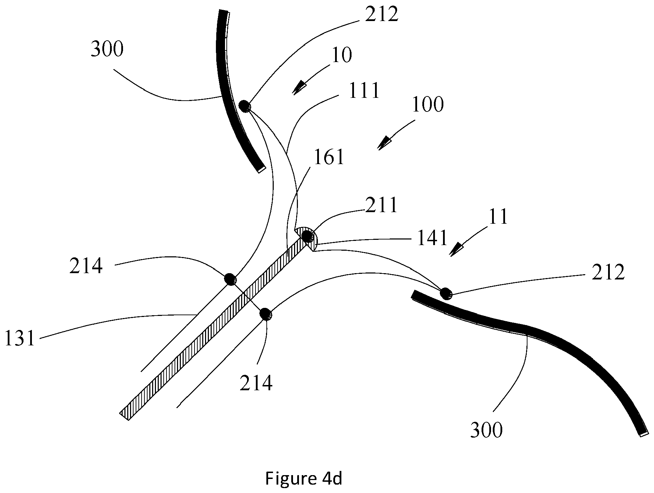

As shown in Fig. 4c, after the occlusion unit 10 is adhered to the atrial septum 300, the distal marker 211 continues to move closer to the atrial septum 300 if the occluder 100 continues to be pulled to the proximal end. When the projection of the distal marker 211 is collinear with the straight line segment, if an acting force is continued to be applied to the occluder 100 to cause the distal marker 211 to continue to move closer to the atrial septum 300, as shown in Fig. 4d, the distal marker 211 may cross the defective position of the atrial septum 300, which results in falling off of the occluder 100. In this way, when the projection of the distal marker 211 is collinear with the straight line segment, a prewarning prompt effect may be achieved for the surgeon to avoid the distal marker 211 from crossing the defective position of the atrial septum 300 and then to avoid the occluder 100 from falling off. Therefore, an exact position and release state of the occluder 100 in the present embodiment can be determined under the DSA to increase the success rate of operation.

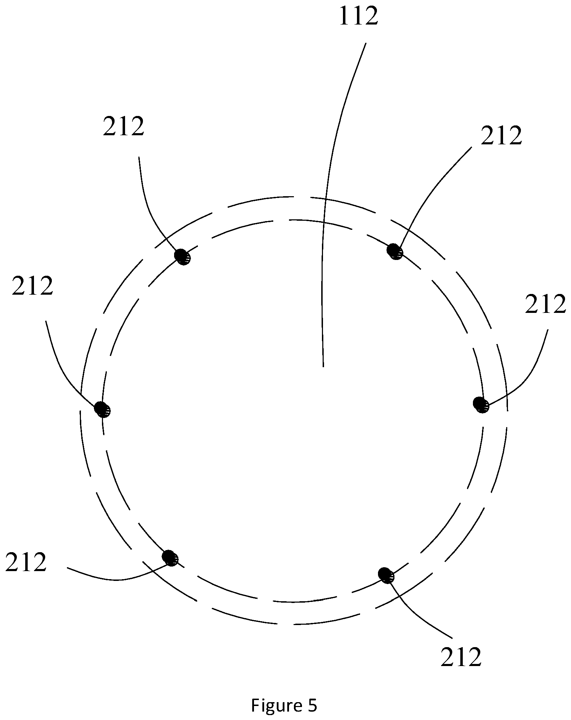

Further, as shown in Fig. 1, the occlusion unit 10 includes a first sub-unit 11, the first sub-unit 11 including a first mesh disk 111 and a first flow blocking film 112 disposed in the first mesh disk 111. The central axis A-A passes through a geometric center of the first mesh disk 111, and the central axis A-A is perpendicular to a plane where the first mesh disk 111 is located. The central axis A-A passes through a geometric center of the first flow blocking film 112 and is perpendicular to a plane where the first flow blocking film 112 is located. All the first markers 212 are connected to the first mesh disk 111. Or, as shown in Fig. 5, in another embodiment, all the first markers 212 are connected to the first flow blocking film 112.

In the present embodiment, the central axis A-A passes through a geometric center of the first mesh disk 111, and the central axis A-A is perpendicular to a plane where the first mesh disk 111 is located such that the plane where the first mesh disk 111 is located is perpendicular to the projection plane. All the first markers 212 are connected to the first mesh disk 111 such that the projections of all the first markers 212 in the projection plane are located on the same straight line segment, and the projection of the distal marker 211 and the connection lines of the two ends of the straight line segment form a triangle. In a similar way, the central axis A-A passes through a geometric center of the first flow blocking film 112, and the central axis A-A is perpendicular to a plane where the first flow blocking film 112 is located such that the plane where the first flow blocking film 112 is located is perpendicular to the projection plane. All the first markers 212 are connected to the first flow blocking film 112 such that the projections of all the first markers 212 in the projection plane are located on the same straight line segment, and further, the projection of the distal marker 211 and the connection lines of the two ends of the straight line segment form a triangle.

As shown in Fig. 4a, in an operation, an acting force is applied to the occluder 100 to cause the first sub-unit 11 to expand in the left atrium. Parameters, such as a probe angle, of the DSA are adjusted to cause the projection points of all the first markers 212 in the projection plane in a picture detected by the DSA to be located on one straight line segment and to cause the connection lines between the distal marker 211 and the two end points of the straight line segment to form a triangle. A surgeon can apply an acting force to the occluder 100 to make it do reciprocating motion. It is observed whether the triangle deforms in the reciprocating motion process. It can be determined that the occlusion unit 10 is not adhered to the atrial septum 300 if the triangle does not deform in this process.

As shown in Fig. 4b, the occlusion unit 10 is continuously applied with an acting force, so that the occlusion unit 10 moves towards the proximal end till the first mesh disk 111 or the first flow blocking film 112 is adhered to the atrial septum 300. In this process, the surgeon observes a geometric shape of the triangle, and is assisted in learning a position relationship between the first mesh disk 111 and the atrial septum 300, so as to facilitate releasing the occluder 100. For example, after the first mesh disk 111 or the first flow blocking film 112 is adhered to the atrial septum 300, the motions of all the first markers 212 are limited, but the distal marker 211 can move freely. At this moment, the distal marker 211 continues to move closer to the atrial septum 300 and the first markers 212, and the triangle may deform. At this moment, it may be determined that the first sub-unit 11 has been adhered to the atrial septum 300.

As shown in Fig. 4c, after the first sub-unit 11 is adhered to the atrial septum 300, the distal marker 211 continues to move closer to the atrial septum 300 if the occluder 100 continues to be pulled to the proximal end. When the projection of the distal marker 211 is collinear with the straight line segment, if an acting force is continued to be applied to the occluder 100 to cause the distal marker 211 to continue to move closer to the atrial septum 300, as shown in Fig. 4d, the distal marker 211 may cross the defective position of the atrial septum 300, which results in falling off of the occluder 100. In this way, when the projection of the distal marker 211 is collinear with the straight line segment, a prewarning prompt effect may be achieved for the surgeon to avoid the distal marker 211 from crossing the defective position of the atrial septum 300 and then to avoid the occluder 100 from falling off and increase the success rate of operation.

In the present embodiment, during use of the occluder 100, the first mesh disk 111 and the first flow blocking film 112 are conveyed to the defect of the atrial septum 300 to occlude the defect of the atrial septum 300. The first mesh disk 111 is made of a material with elastic memory. When the first mesh disk 111 is conveyed to a desired position and moves out of the sheath 400, the first mesh disk 111 can expand under the action of its own elastic force and restore to a natural state, and when restored to the natural state, the first mesh disk 111 can drive the first flow blocking film 112 to expand, so that the first mesh disk 111 and the first flow blocking film 112 occlude the defect of the atrial septum 300.

It can be understood that, as shown in Fig. 1, all the first markers 212 are fixed on the first mesh disk 111. When the occluder 100 is in a natural state, the distance between each first marker 212 and the central axis A-A is equal to the distance from an edge of the first mesh disk 111 to a geometric center of the first mesh disk 111.

In the present embodiment, in an operation, when the edge of the first mesh disk 111 is adhered to the atrial septum 300, all the first markers 212 are fixed on the first mesh disk 111, and the distance between each first markers 212 and the central axis A-A is equal to the distance from an edge of the first mesh disk 111 to the geometric center of the first mesh disk 111, so that it can be ensured that all the first markers 212 are adhered to the atrial septum 300, and the accuracy for guiding the operation by the cooperative use of the distal marker 211 and the first markers 212 can be improved.

In one embodiment, the first mesh disk 111 is circular. The central axis A-A passes through a circle center of the first mesh disk 111 (i.e., the geometric center of the first mesh disk 111), and all the first markers 212 are fixed on the circumference of the first mesh disk 111. The distance between each first marker 212 and the circle center of the first mesh disk 111 is equal to a radius of the first mesh disk 111.

Of course, in other embodiments, when the occluder 100 is in a natural state, the distance between each first marker 212 and the central axis A-A may also be less than the distance from the edge of the first mesh disk 111 to the geometric center of the first mesh disk 111. For example, the first mesh disk 111 is circular. The central axis A-A passes through a circle center of the first mesh disk 111 (i.e., the geometric center of the first mesh disk 111), and all the first markers 212 are fixed on the first mesh disk 111 along a circumference. The distance between each first marker 212 and the circle center of the first mesh disk 111 is equal to a radius of the first mesh disk 111. For example, the diameter of the circumference where all the first markers 212 are located is less than the diameter of the first mesh disk 111 by 1 to 8 mm. In the present embodiment, the distance between each first marker 212 and the central axis A-A may also be less than the distance from the edge of the first mesh disk 111 to the geometric center of the first mesh disk 111, so as to facilitate machining. In addition, when the occluder 100 is conveyed out of the sheath 400, the first markers 212 can be avoided from generating friction with an inner wall of the sheath 400 may also be avoided, so as to avoid the first markers 212 from falling off.

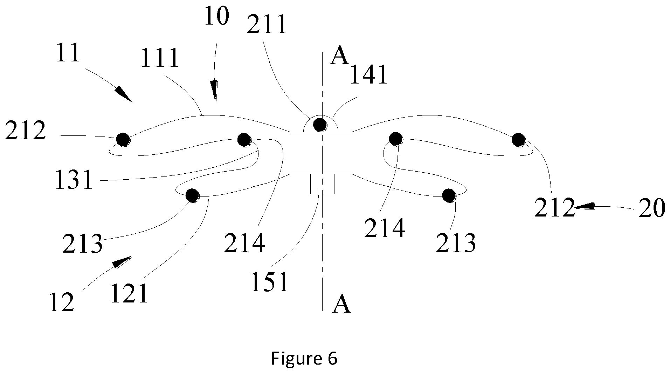

It can be understood that all the first markers 212 are fixed on the first flow blocking film 112. When the occluder 100 is in a natural state, the distance between at least one of the first markers 212 and the central axis A-A is equal to a distance from an edge of the first flow blocking film 112 to a geometric center of the first flow blocking film 112. Or, as shown in Fig. 6, the distance between each first marker 212 and the central axis A-A is less than the distance from the edge of the first flow blocking film 112 to the geometric center of the first flow blocking film 112.

In the present embodiment, when the distance between each first marker 212 and the central axis A-A is equal to the distance from the edge of the first flow blocking film 112 to the geometric center of the first flow blocking film 112, for example, when the first flow blocking film 112 is circular, the central axis A-A passes through a circle center of the first flow blocking film 112 (i.e., the geometric center of the first flow blocking film 112). All the first markers 212 are fixed at the edge of the first flow blocking film 112, and the distance from each first marker 212 to the circle center of the first flow blocking film 112 is equal to a radius of the first flow blocking film 112. In an operation, the accuracy for guiding the operation by the cooperative use of the distal marker 211 and the first markers 212 can be improved. When the distance between each first marker 212 and the central axis A-A is less than the distance from the edge of the first flow blocking film 112 to the geometric center of the first flow blocking film 112, all the first markers 212 can be avoided from generating friction with the inner wall of the sheath 400, so as to avoid the first markers 212 from falling off.

For example, in one embodiment, when the occluder 100 is in a natural state, the distance between each first marker 212 and the central axis A-A is less than the distance from the edge of the first flow blocking film 112 to the geometric center of the first flow blocking film 112, and all the first markers 212 are distributed along the first circumference. The first circumference and the first flow blocking film 112 are concentrically disposed.

In the present embodiment, the distance between each first marker 212 and the central axis A-A is less than the distance from the edge of the first flow blocking film 112 to the geometric center of the first flow blocking film 112. For example, the first flow blocking film 112 is circular, and the central axis A-A passes through the circle center of the first flow blocking film 112 (i.e., the geometric center of the first flow blocking film 112); and all the first markers 212 are fixed on the first flow blocking film 112 along the first circumference, and the diameter of the first circumference is less than the diameter of the first flow blocking film by 1 to 8 mm. All the first markers 212 can be avoided from generating friction with the inner wall of the sheath 400, so as to avoid the first markers 212 from falling off.

It can be understood that all the first markers 212 are distributed equidistantly along the first circumference, and the number of the first markers 212 ranges from 2 to 12.

For example, as shown in Fig. 5, in one embodiment, the marking structure 20 includes six first markers 212, and the six first markers 212 are distributed along the first circumference at equal intervals.

It can be understood that, as shown in Fig. 1, the occlusion unit 10 further includes a second sub-unit 12 connected to the first sub-unit 11. The second sub-unit 12 includes a second mesh disk 121 and a second flow blocking film 122 disposed in the second mesh disk 121. The central axis A-A passes through a geometric center of the second mesh disk 121, and the central axis A-A is perpendicular to a plane where the second mesh disk 121 is located. The central axis A-A passes through a geometric center of the second flow blocking film 122, and the central axis A-A is perpendicular to a plane where the second flow blocking film 122 is located. The marking structure 20 further includes at least two second markers 213. All the second markers 213 are connected to the second mesh disk 121, or, all the second markers 213 are connected to the second flow blocking film 122.

In the present embodiment, the central axis A-A passes through the geometric center of the second mesh disk 121, and the central axis A-A is perpendicular to the plane where the second mesh disk 121 is located, so that the plane where the second mesh disk 121 is perpendicular to the projection plane. All the second markers 213 are connected to the second mesh disk 121, so that projections of all the second markers 213 in the projection plane are located on the same straight line segment. In a similar way, the central axis A-A passes through the geometric center of the second flow blocking film 122, and the central axis A-A is perpendicular to the plane where the second flow blocking film 122 is located, so that the plane where the second flow blocking film 122 is perpendicular to the projection plane. All the second markers 213 are connected to the second flow blocking film 122, so that projections of all the second markers 213 in the projection plane are located on the same straight line segment.

It can be understood that all the second markers 213 are fixed on the second mesh disk 121. When the occluder 100 is in a natural state, as shown in Fig. 1, the distance between each second marker 213 and the central axis A-A is equal to a distance from an edge of the second mesh disk 121 to a geometric center of the second mesh disk 121. In the present embodiment, when the occluder 100 is in a natural state, the distance between each second marker 213 and the central axis A-A is equal to the distance from the edge of the second mesh disk 121 to the geometric center of the second mesh disk 121. In an operation, when the edge of the second mesh disk 121 is adhered to the atrial septum 300, it can be ensured that all the second markers 213 are adhered to the atrial septum 300, and the accuracy for guiding the second markers 213 can be improved.

It can be understood that the central axis A-A passes through the geometric center of the second mesh disk 121, and the central axis A-A is perpendicular to the plane where the second mesh disk 121 is located. All the second markers 213 are fixed on the second mesh disk 121. When the occluder 100 is in a natural state, the distance between each second marker 213 and the central axis A-A is less than the distance from the edge of the second mesh disk 121 to the geometric center of the second mesh disk 121. In the present embodiment, when the occluder 100 is in a natural state, the distance between each second marker 213 and the central axis A-A is less than the distance from the edge of the second mesh disk 121 to the geometric center of the second mesh disk 121. When the second mesh disk 121 is located in the sheath 400, the second markers 213 may be avoided from generating friction with the inner wall of the sheath 400, so as to avoid the second markers 213 from falling off.

It can be understood that all the second markers 213 are fixed on the second flow blocking film 122. When the occluder 100 is in a natural state, the distance between at least one of all the second markers 213 and the central axis A-A is equal to the distance from the edge of the second flow blocking film 122 to the geometric center of the second flow blocking film 122, or the distance between each second marker 213 and the central axis A-A is equal to the distance from the edge of the second flow blocking film 122 to the geometric center of the second flow blocking film 122.

For example, when the occluder 100 is in a natural state, the distance between each second marker 213 and the central axis A-A is less than the distance from the edge of the second flow blocking film 122 to the geometric center of the second flow blocking film 122, and all the second markers 213 are distributed along a second circumference. The second circumference and the second flow blocking film 122 are concentrically disposed. In the present embodiment, the distance between each second marker 213 and the central axis A-A is less than the distance from the edge of the second flow blocking film 122 to the geometric center of the second flow blocking film 122, and all the second markers 213 are located on a second circumference. When the second flow blocking film 122 is located in the sheath 400, the second markers 213 can be avoided from generating friction with the inner wall of the sheath 400, so as to avoid the second markers 213 from falling off.

For example, all the second markers 213 are distributed along the second circumference at equal intervals, and the number of the second markers 213 ranges from 2 to 12.

It can be understood that, as shown in Fig. 1, the occlusion unit 10 further includes a waist portion 131 and a third flow blocking film 132. Two ends of the waist portion 131 are respectively connected to the first mesh disk 111 and the second mesh disk 121. The third flow blocking film 132 is disposed in the waist portion 131. The marking structure 20 further includes at least two third markers 214. The central axis A-A passes through a geometric center of the third flow blocking film 132, and the central axis A-A is perpendicular to a plane where the third flow blocking film 132 is located. All the third markers 214 are connected to the waist portion 131, or, all the third markers 214 are connected to the third flow blocking film 132.

In the present embodiment, the central axis A-A passes through the geometric center of the third flow blocking film 132, and the central axis A-A is perpendicular to the plane where the third flow blocking film 132 is located, so that the plane where the third flow blocking film 132 is located may be perpendicular to the projection plane. All the third markers 214 are connected to the third flow blocking film 132, so that projections of all the second markers 213 in the projection plan are located on the same straight line segment. In an operation, when the first sub-unit 11 reaches a desired position (i.e., the first sub-unit 11 is adhered to the atrial septum 300), parameters such as the probe angle of the DSA are adjusted to cause a straight line formed by the third markers 214 in the projection plane and a straight line formed by the first markers 212 to be parallel to each other, and a surgeon can determines a relative position between the third markers 214 and the atrial septum 300 according to the size of a distance between the two straight lines, so as to determine relative positions between the waist portion 131 as well as between the third flow blocking film 132 and the atrial septum 300.

It can be understood that all the third markers 214 are connected with the third flow blocking film 132. When the occluder 100 is in a natural state, a distance between at least one of all the third markers 214 and the central axis A-A is equal to a distance from an edge of the third flow blocking film 132 to a geometric center of the third flow blocking film 132, or, the distance between each third marker 214 and the central axis A-A is less than the distance from the edge of the third flow blocking film 132 to the geometric center of the third flow blocking film 132.

In the present embodiment, when the distance between each third marker 214 and the central axis A-A is equal to the distance from the edge of the third flow blocking film 132 to the geometric center of the third flow blocking film 132, it contributes to improving the accuracy of the third markers 214 for guiding an operation. When the distance between each third markers 214 and the central axis A-A is less than the distance from the edge of the third flow blocking film 132 to the geometric center of the third flow blocking film 132, the third markers 214 may be avoided from generating friction with the inner wall of the sheath 400 in a release process of the waist portion 131, and the third markers 214 may be prevented from falling off.

In one embodiment, the third flow blocking film 132 is circular. The central axis A-A passes through a circle center of the third flow blocking film 132 (i.e., the central axis A-A passes through the geometric center of the third flow blocking film 132), and the central axis A-A is perpendicular to a plane where the third flow blocking film 132 is located. All the third markers 214 are fixed on the third flow blocking film 132 along a circumference, and the diameter of the circumference where all the third markers 214 are located is less than the diameter of the third flow blocking film 132 by 1 to 8 mm. In the release process of the waist portion 131, the third markers 214 may be avoided from generating friction with the inner wall of the sheath 400, and the third markers 214 may be prevented from falling off.

Of course, in another embodiment, the waist portion 131 is circular. The central axis A-A passes through a circle center of the waist portion 131 (i.e., the central axis A-A passes through the geometric center of the waist portion 131). All the third markers 214 are fixed on the waist portion 131 along a circumference, and the diameter of the circumference where all the third markers 214 are located is less than the diameter of the waist portion 131 by 1 to 8 mm. In the release process of the waist portion 131, the third markers 214 may be avoided from generating friction with the inner wall of the sheath 400, and the third markers 214 may be prevented from falling off.

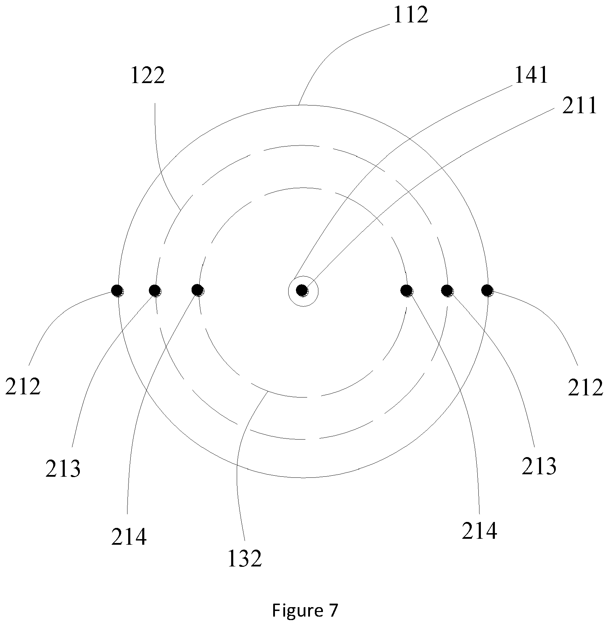

For example, as shown in Fig. 7, in one embodiment, the number of the first markers 212, the number of the second markers 213 and the number of the third markers 214 are all equal to 2. When the occluder 100 is in a natural state, the two first markers 212 are located at two ends of the same diameter of the first flow blocking film 112, the two second markers 213 are located at two ends of the same diameter of the second flow blocking film 122, and the two third markers 214 are located at two ends of the same diameter of the third flow blocking film 132; and the two first markers 212, the two second markers 213 and the two third markers 214 are coplanar.
In the present embodiment, the two first markers 212, the two second markers 213 and the two third markers 214 are coplanar, which contributes to improving the precision for determining the forms of the first mesh disk 111, the second mesh disk 121 and the waist portion 131.

As shown in Fig. 1, the occluder 100 further includes a bolt head 151. The bolt head 151 is fixed at the proximal end of the occlusion unit 10. The conveyor further includes a conveying head made of a developing material, and the conveying head is in threaded connection with the bolt head 151.

It can be understood that as shown in Fig. 1 and Fig. 4a, the occluder 100 further includes a locking pin 161. The locking pin 161 is connected between the occlusion head 141 and the bolt head 151. The marking structure 20 further includes a proximal marker arranged on the bolt head 151. Whether the occluder 100 and the conveyor are separated successfully can be determined according to a relative position between the proximal marker and the conveying head.

It can be understood that in a process of releasing the first mesh disk 111, the first flow blocking film 112 is released together with the first mesh disk 111. Similarly, in a process of releasing the second mesh disk 121, the second flow blocking film 122 is released together with the second mesh disk 121. When the waist portion 131 is released, the third flow blocking film 132 is released together with the waist portion 131.

It can be understood that the first mesh disk 111, the second mesh disk 121, and the waist portion 131 can be prepared in a manner of weaving, cutting and the like. Weaving is taken as an example. A material of a weaving filament may be a non-degradable metal material such as a NiTi alloy, a Co-Cr alloy and stainless steel, and may also be made of a biodegradable material.

When the first mesh disk 111, the second mesh disk 121, and the waist portion 131 are made of the biodegradable material, the biodegradable material includes, but is not limited to, polylactic acid (PLA), poly-DL-lactic acid (PDLLA), polyglycolic acid (PGA), polylactic-glycolic acid copolymer (PLGA), polyhydroxyalkanoate (PHA), polydioxanone (PDO), or polycaprolactone (PCL), and the like. The woven first mesh disk 111, second mesh disk 121, and waist portion 131 are thermally treated through a mold and set into predetermined shapes.

A material of the marking structure 20 may be a metal substance, such as gold, platinum, barium sulfate, etc., or a non-metal substance such as sodium bromide, sodium iodide, iohexol, iodide, etc. When the first markers 212 are fixed on the first mesh disk 111, the first markers 212 are fixed at grid intersection points of the first mesh disk 111, or wound on weaving monofilaments, or welded on the weaving monofilaments, or coated on surfaces of the weaving monofilaments. By the above setting mode of the marking structure 20, the structure is simple, firm, and reliable, and does not affect the overall structure of the occluder 100.

It can be understood that when the second markers 213 are fixed on the second mesh disk 121 or the above-mentioned fixing mode can be referred. When the third markers 214 are fixed on waist portion 131, the above-mentioned fixing mode can also be referred. No repeated descriptions are provided here.

It can be understood that when the first markers 212 are fixed on the first flow blocking film 112, the fixing mode may be embedding, adhesion, suturing, and the like.

All features of the above-mentioned embodiments may be combined in any combination. In order to simplify the description, all possible combinations of all the features in the above-mentioned embodiments are not described. However, insofar as the combinations of these features do not contradict, they should be considered to be the scope of the embodiments described.

The above embodiments represent only a few potential embodiments, and are described in more detail but are not to be construed as limiting. It should be noted that those of ordinary skill in the art can also made numerous variations and modifications without departing from the concept of the embodiments, and these variations and modifications shall all fall within the protection scope of the embodiments.

## Claims

1. An occluder, comprising: an occlusion unit and an occlusion head connected to the occlusion unit, the occlusion unit having a central axis passing through a distal end and a proximal end of the occluder, the occluder further comprising a marking structure, the marking structure comprising a distal marker and at least two first markers; wherein
the distal marker is disposed on the occlusion head;
all the first markers are connected to the occlusion unit;
all the first markers are located in the same placement plane;
the placement plane is perpendicular to the central axis;
the distal marker is located outside the placement plane;
projection points of all the first markers in a projection plane perpendicular to the placement plane are located on a straight line segment; and
when the occluder is in a natural state, connection lines between the distal marker and two end points of the straight line segment form a triangle.

2. The occluder according to claim 1, wherein the occlusion unit comprises a first sub-unit; the first sub-unit comprises a first mesh disk and a first flow blocking film disposed in the first mesh disk;
the central axis passes through a geometric center of the first mesh disk, and the central axis is perpendicular to a plane where the first mesh disk is located;
the central axis passes through a geometric center of the first flow blocking film, and the central axis is perpendicular to a plane where the first flow blocking film is located; and
all the first markers are connected to the first mesh disk, or all the first markers are connected to the first flow blocking film.

3. The occluder according to claim 2, wherein all of the first markers are fixed on the first mesh disk; and
when the occluder is in the natural state, a distance between each first marker and the central axis is equal to or less than a distance from an edge of the first mesh disk to the geometric center of the first mesh disk.

4. The occluder according to claim 2, wherein all of the first markers are fixed on the first flow blocking film; and
when the occluder is in the natural state, a distance between at least one of the first markers and the central axis is equal to a distance from an edge of the first flow blocking film to the geometric center of the first flow blocking film, or a distance between each first marker and the central axis is less than a distance from the edge of the first flow blocking film to the geometric center of the first flow blocking film.

5. The occluder according to claim 4, wherein, when the occluder is in the natural state, the distance between each first marker and the central axis is less than the distance from the edge of the first flow blocking film to the geometric center of the first flow blocking film;
all the first markers are distributed along a first circumference; and
the first circumference and the first flow blocking film are concentrically disposed.

6. The occluder according to claim 5, wherein all of the first markers are distributed equidistantly along the first circumference, and a number of the first markers ranges from 2 to 12.

7. The occluder according to claim 2, wherein the occlusion unit further comprises a second sub-unit connected to the first sub-unit;
the second sub-unit comprises a second mesh disk and a second flow blocking film disposed in the second mesh disk;
the central axis passes through a geometric center of the second mesh disk, and the central axis is perpendicular to a plane where the second mesh disk is located;
the central axis passes through a geometric center of the second flow blocking film, and the central axis is perpendicular to a plane where the second flow blocking film is located;
the marking structure further comprises at least two second markers; and
all of the second markers are connected to the second mesh disk, or all of the second markers are connected to the second flow blocking film.

8. The occluder according to claim 7, wherein all of the second markers are fixed on the second mesh disk; and
when the occluder is in the natural state, a distance between each second marker and the central axis is equal to or less than a distance from an edge of the second mesh disk to the geometric center of the second mesh disk.

9. The occluder according to claim 7, wherein all the second markers are fixed on the second flow blocking film; and
when the occluder is in the natural state, a distance between at least one of the second markers and the central axis is equal to a distance from an edge of the second flow blocking film to the geometric center of the second flow blocking film, or a distance between each second marker and the central axis is less than a distance from the edge of the second flow blocking film to the geometric center of the second flow blocking film.

10. The occluder according to claim 9, wherein when the occluder is in the natural state, the distance between each second marker and the central axis is less than the distance from the edge of the second flow blocking film to the geometric center of the second flow blocking film;
the second markers are distributed along a second circumference; and
the second circumference and the second flow blocking film are concentrically disposed.

11. The occluder according to claim 10, wherein the second markers are distributed equidistantly along the second circumference, and a number of the second markers ranges from 2 to 12.

12. The occluder according to claim 7, wherein the occlusion unit further comprises a waist portion and a third flow blocking film;
two ends of the waist portion are respectively connected to the first mesh disk and the second mesh disk;
the third flow blocking film is disposed in the waist portion;
the central axis passes through a geometric center of the third flow blocking film, and the central axis is perpendicular to a plane where the third flow blocking film is located;
the marking structure further comprises at least two third markers; and
all the third markers are connected to the waist portion, or all of the third markers are connected to the third flow blocking film.

13. The occluder according to claim 12, wherein all of the third markers are connected to the third flow blocking film; and
when the occluder is in the natural state, a distance between at least one of the third markers and the central axis is equal to a distance from an edge of the third flow blocking film to a geometric center of the third flow blocking film, or, a distance between each third marker and the central axis is less than a distance from the edge of the third flow blocking film to the geometric center of the third flow blocking film.

14. The occluder according to claim 12, wherein the number of the first markers, the number of the second markers and the number of the third markers are all equal to 2; and,
when the occluder is in the natural state, the two first markers are located at two ends of a same diameter of the first flow blocking film, the two second markers are located at two ends of a same diameter of the second flow blocking film, and the two third markers are located at two ends of a same diameter of the third flow blocking film; and the two first markers, the two second markers and the two third markers are coplanar.
